(19)

Europäisches
Patentamt
European
Patent Office
Office européen
des brevets

(11) **EP 2 279 761 B1**

(12) **EUROPEAN PATENT SPECIFICATION**

(45) Date of publication and mention
of the grant of the patent:
**23.05.2012 Bulletin 2012/21**

(51) Int Cl.:
*A61L 2/14* *(2006.01)*    *A61L 2/20* *(2006.01)*
*A61L 101/22* *(2006.01)*

(21) Application number: **10179975.7**

(22) Date of filing: **30.03.2007**

(54) **A method for prion inactivation by hydrogen peroxide**

Verfahren zur Prioneninaktivierung durch Wasserstoffperoxid

Procédé d'inactivation de prion par peroxyde d'hydrogène

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **31.03.2006 US 395604**
**31.10.2006 US 554849**

(43) Date of publication of application:
**02.02.2011 Bulletin 2011/05**

(62) Document number(s) of the earlier application(s) in
accordance with Art. 76 EPC:
**07251396.3 / 1 839 679**

(73) Proprietor: **Ethicon, Incorporated**
**Somerville, NJ 08876 (US)**

(72) Inventors:
• **Roberts, Charles, G.**
**Long Beach, CA 90815 (US)**
• **Favero, Martin**
**San Clemente, CA 92673 (US)**

(74) Representative: **Fisher, Adrian John**
**Carpmaels & Ransford**
**One Southampton Row**
**London**
**WC1B 5HA (GB)**

(56) References cited:
**WO-A-03/031551**    **WO-A1-02/07788**
**US-A1- 2002 114 727**    **US-A1- 2006 008 494**

• **BLOCK, S.S.: "Sterilization, disinfection, and
preservation, 5TH Edition" 31 December 2001
(2001-12-31), LIPPINCOTT WILLIAMS &
WILKINS , XP002606842page 750-757, Marked
passages**
• **FICHET G ET AL: "Novel methods for disinfection
of prion-contaminated medical devices" LANCET
THE, LANCET LIMITED. LONDON, GB, vol. 364,
no. 9433, 7 August 2004 (2004-08-07), pages
521-526, XP004738179 ISSN: 0140-6736**

**Description**

FIELD OF THE INVENTION

The invention relates to treatment of articles, and more particularly to treatment of articles to inactivate prions thereon.

BACKGROUND OF THE INVENTION

WO03/031551 discloses a method for prion decontamination using vaporous hydrogen peroxide, which might have a concentration up to 95 %

US2006/008494 discloses a liquid formulation for prion decontamination comprising *inter alia* H2O2.

Most medical instruments are sterilized prior to u e. Most are sterilized and sold in protective packaging to maintain their sterile state until the package is opened and the instrument is used in a medical procedure. Some instruments are re-used and must be sterilized prior to such re-use. Common methods of sterilization include high temperature steam, irradiation, and chemical vapors such as ethylene oxide and hydrogen peroxide.

Prions are proteinacious infectious agents which cause similar fatal brain diseases, known as transmissible spongiform encephalopathies (TSEs). These diseases include Creutzfeldt-Jakob disease (CJD) in humans, Bovine Spongiform Encephalopathy (BSE) in cattle, also known as "mad cow" disease, Scrapie in sheep, and Wasting disease in elk and deer. Creutzfeldt-Jakob variant disease (CJDv) in humans is believed to be caused by the same proteins which cause BSE in cattle. The prions are believed to be an abnormal form of a protein commonly found in the host. However, unlike the normal form of the protein, the

abnormal proteins accumulate, particularly in neural tissue, eventually causing neural cell death.

Common sterilization methods have not been successful in rendering prions non-infectious. Given that prions contain no DNA or RNA they are typically not considered alive and cannot be killed in the conventional sense in which one thinks of sterilization of articles by killing infections microorganisms thereon. Rather, the goal is to inactivate the prion in some manner, such as by breaking one or more of its chemical bonds, to leave it unable to cause prion disease such as CJD or CJDv in a host.

The generally accepted methodology for inactivating prions involves treatment with solutions having extremely high pH and temperature in an effort to denature the prion. The manner in which a prion is folded tends to make denaturing the prion using typical protein denaturing agents ineffective. The elevated temperature and pH of the accepted methodology is often quite damaging to the instruments.

It had been thought that sterilization systems which employ hydrogen peroxide vapor and or hydrogen peroxide vapor coupled with plasma would be unable to inactivate prions. Tests were run on a typical system in which a 59% solution of hydrogen peroxide is admitted into a sterilization chamber under vacuum conditions to create an effective sterilizing vapor of hydrogen peroxide and in which the vapor is at one point excited into the plasma stage. This methodology failed to sufficiently inactivate prions on the instruments to render them non-infectious.

Surprisingly, the present inventors have found that application of hydrogen peroxide vapor of higher concentrations and/or higher plasma density is effective in inactivating prions and rendering an article so treated non-infections for prion diseases.

SUMMARY OF THE INVENTION

A method of inactivating prions according to the present invention comprises the steps of: placing an article having a quantity of prions thereon into a sterilization chamber; lowering pressure in the sterilization chamber to a pressure below the vapor pressure of hydrogen peroxide in the chamber; vaporizing a solution comprising hydrogen peroxide and contacting the article with hydrogen peroxide vapor; and inactivating prions in the chamber through contact between the prions and the hydrogen peroxide vapor.

The concentration of the hydrogen peroxide in the solution exceeds 70 percent, more preferably 75 percent, more preferably 80 percent, more preferably 85 percent, and most preferably exceeds 90 percent.

Preferably, the temperature of the article during the step of contacting the article with hydrogen peroxide vapor exceeds 30 degrees Celsius, more preferably 35 degrees Celsius, more preferably 40 degrees Celsius, and most preferably exceeds 45 degrees Celsius.

The method further comprises exciting the hydrogen peroxide vapor into the plasma phase and contacting the prions with the hydrogen peroxide in the plasma phase. The plasma density is at least 10 milliwatts/cm$^3$, more preferably 20 milliwatts/cm$^3$, more preferably 30 watts/cm$^3$, more preferably 40 watts/cm$^3$, and most preferably at least 50 milliwatts/cm$^3$.

Preferably, the plasma is in contact with the prions for at least 2 minutes, more preferably 3 minutes, and most preferably at least 4 minutes.

Preferably, the plasma is generated between an anode and a cathode and wherein a gap therebetween is less than 1.5cm (0.6 inches), perhaps less than 1.3 cm (0.5 inches). A gap of about 1.3cm (0.5 inches) is also preferred. Preferably, a minimum distance between the prions and the closer of the anode and cathode is 3.8cm (1.5 inches), more preferably 2.5cm (one inch). The minimum distance between the prions and the anode may be one inch or less.

Preferably, the hydrogen peroxide also effects sterilization of the article.

The concentration of at least a portion of the hydrogen peroxide can be increased prior to contacting the prions therewith, preferably by selectively removing water from the solution and exhausting that water through the sterilization chamber.

Gases surrounding the prions can be excited into the plasma phase and then contacted with the prions.

The steps of steps of lowering pressure in the sterilization chamber and admitting the solution into the sterilization chamber comprise a cycle and wherein the cycle is repeated one or more times.

Preferably, the hydrogen peroxide vapor which contacts the article has a concentration of 10 mg/L or more, preferably 15 mg/L or more, preferably 20 mg/L or more, most preferably 25 mg/L or more.

Preferably, the method further includes the step of condensing a portion of the hydrogen peroxide vapor onto the article and wherein the concentration of that condensed hydrogen peroxide is at least 80% hydrogen peroxide by weight, more preferably at least 85%, more preferably at least 90% and most preferably at least 95%. Preferably at least a portion, if not all, of the hydrogen peroxide which has condensed onto the article is re-vaporized by pumping the chamber down to a lower pressure.

BRIEF DESCRIPTION OF THE DRAWINGS

FIG. 1 is a block diagram of a sterilization system according to the present invention;

FIG. 2 is a block diagram of a vaporizer and diffusion path of the sterilization system of FIG. 1;

FIG. 3 is a block diagram of an alternate embodiment of a sterilization system according to the present invention;

FIG. 3A is a block diagram of an alternative embodiment of a sterilization system according to the present invention.

FIG. 3B is a sectional view taken along lines 3B--3B of FIG 3A;

FIG. 4 is a block diagram of an alternate embodiment of a sterilization system according to the present invention;

FIG. 5 is a block diagram of an alternate embodiment of a sterilization system according to the present invention;

FIG. 6 is a section view taken along lines 6--6 of FIG. 5;

FIG. 7 is a block diagram of an alternate embodiment of a sterilization system according to the present invention;

FIG. 8 is a section view taken along lines 8--8 of FIG. 7;

FIG. 9 is a block diagram of a sterilization system according to the present invention;

FIG. 10 is a cut-away view of an outlet condenser/vaporizer for use in the system of FIG. 9;

FIG. 11 is a cut-away view of an inlet condenser/vaporizer for use in the system of FIG. 9;

FIG. 12 is a perspective view of an alternative inlet condenser/vaporizer for use in the system of FIG. 9;

FIG. 13 is an exploded perspective view of the condenser/vaporizer of FIG. 12;

FIG. 14 is a section view taken along lines 14--14 of FIG. 12;

FIG. 14A is a close-up section view of the valve assembly shown in FIG. 14;

FIG. 15 is an exploded perspective view of a thermoelectric heat pump and rod assembly employed in the condenser/vaporizer of FIG. 12;

FIG. 16 is an alternative sterilization system according to the present invention;

FIG. 17 is an alternative sterilization system according to the present invention;

FIG. 18 is an alternative sterilization system according to the present invention;

FIG. 19 is an alternative sterilization system according to the present invention;

FIG. 20 is a perspective view of an alternative inlet condenser/vaporizer for use in the system of FIG. 9;

FIG. 21 is a valve block employed in the inlet condenser/vaporizer of FIG. 20;

FIG. 22 is a cut-away view of the valve block of FIG. 21 as employed in the inlet condenser/vaporizer of FIG. 20; and

FIG. 23 is a flow diagram for calculating the concentration of peroxide which condenses upon the load.

## DETAILED DESCRIPTION

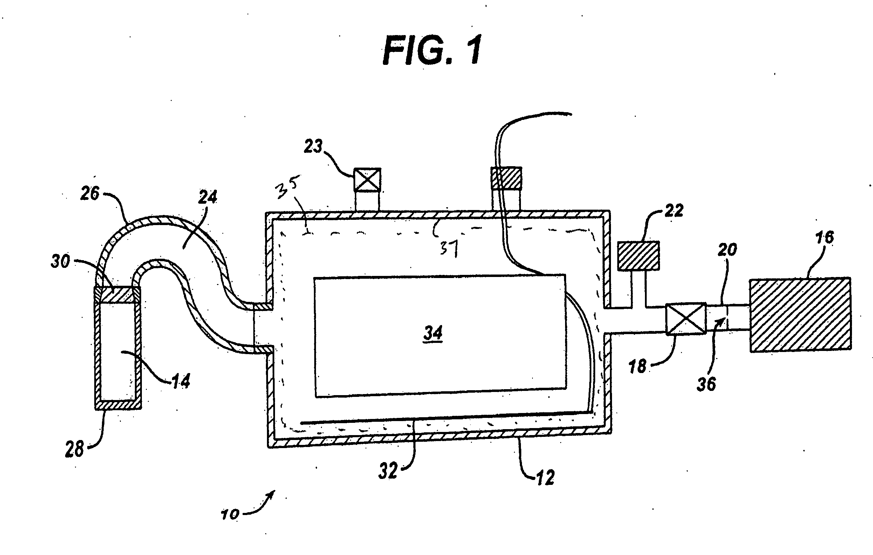

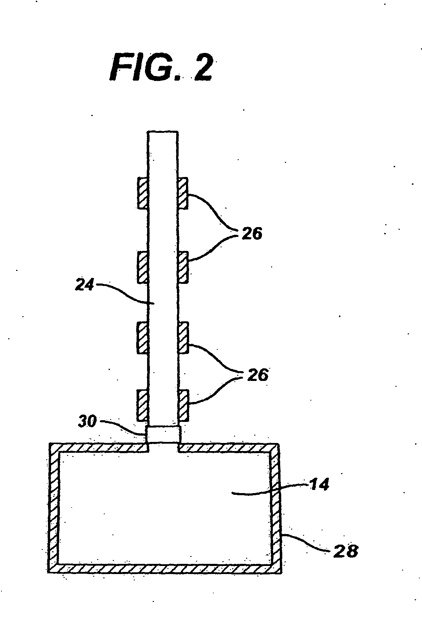

FIG. 1 shows in block diagram form a sterilization system 10 which is effective not only in killing traditional infectious microorganisms such as bacteria and viruses, but has also been shown to be effective in inactivating prions. It comprises in gross a sterilization chamber 12, a vaporizer 14, and a vacuum pump 16. The vacuum pump is capable of drawing a vacuum on the chamber, preferably as low as 0.5 torr. Between the vacuum pump 16 and the chamber 12, is preferably located at throttle valve 18 and optionally an orifice plate 20. The throttle valve 18 preferably also has good shut-off capability. A pressure gauge 22, preferably located adjacent to the throttle valve 18, shows the vacuum in the chamber 12. A vent valve 23 employing a HEPA antimicrobial filter allows clean sterile air to enter the chamber 12. The vaporizer 14 connects to the chamber 12 by means of an elongated diffusion path 24. Turning also to FIG. 2, the diffusion path 24 incorporates temperature control elements 26 to control the temperature along the diffusion path 24.

Vaporizers suitable for vaporizing a liquid sterilant such as hydrogen peroxide solution are known in the art. Kohler et al. U.S. Patent No. 6,106,772 and Nguyen et al. U.S. Patent Application No. 09/728,973 filed December 10, 2000, illustrate vaporizers suitable for the present application. In its simplest for the vaporizer can comprise a small chamber into which the liquid hydrogen peroxide solution is injected. The low pressure in the vaporizer caused by the vacuum in the chamber causes the hydrogen peroxide solution to vaporize.

Preferably, the vaporizer 14 itself incorporates heating elements 28 which control the temperature in the vaporizer to optimize the vaporization process. Preferably, where the vaporizer 14 connects to the diffusion path 24 some form of thermal insulation 30 provided at the interface so that the high temperatures of the vaporizer 14 will not unduly affect the temperature in the diffusion path 24. The vaporizer 14 and diffusion path 24 are preferably formed of aluminum; the thermal insulation 30 can take the form of a polyvinyl chloride (PVC) joint connecting the two together.

Further, it is preferable to include a heater 32 inside the chamber 12, preferably near a lower portion of the chamber 12 for revaporizing condensed hydrogen peroxide inside the chamber 12.

The chamber 12 preferably includes a mechanism (not shown) to create a plasma therein. Such mechanism can include a source of radio or low frequency energy as described by Jacobs et al. U.S. Patent No. 4,643,867,or by Platt, Jr. et al. in published U.S. Application Document No. 20020068012, both of which are incorporated herein by reference.

The present invention achieves its beneficial effect by allowing some of the hydrogen peroxide which is vaporized out of solution in the vaporizer 14 to condense onto the diffusion path 24. After most of the hydrogen peroxide solution has vaporized, the temperature control elements 26 raise the temperature of the diffusion path to allow the condensed hydrogen peroxide to re-vaporize. Water has a higher vapor pressure than hydrogen peroxide, thus hydrogen peroxide in the vapor condenses more easily than water. Thus, the material which condenses in the diffusion path will have a higher concentration of hydrogen peroxide than the starting concentration of the hydrogen peroxide solution in the vaporizer 14.

The temperature control elements 26 in simple form can comprise mere electric resistance heaters. In such case, the low ambient temperature of the diffusion path 24 provides the low temperature for condensing hydrogen peroxide thereon, and the control elements 26 later heat the diffusion path 24 to re-vaporize the now more highly concentrated hydrogen peroxide from the diffusion path 24. Because the vapor pressure of hydrogen peroxide drops with lower temperatures, lower initial temperatures in the diffusion path 24 allows a lower pressure in the chamber 24 without subsequently preventing the condensation of hydrogen peroxide in the diffusion path. Lower chamber pressures promote system efficiency and thus, the temperature control elements 26 can further comprise a chilling component to lower the temperature of the diffusion path below ambient. Suitable chilling components include thermoelectric coolers or a typical mechanical refrigeration system. In such case, the diffusion path 24 would be first chilled, preferably to about 10°C, and then some time after vaporization has begun or even after it has completed, the diffusion path 24 is then heated, preferably up to 50°C or 110°C.

When vertically oriented as in FIG. 2, the diffusion path 24 can potentially cause the vaporizing sterilant to condense in cooler regions between the temperature control elements 26 and then re-vaporize as it passes the temperature control element 26.

The following example illustrates the benefits of controlling the heat in the diffusion path.

EXAMPLE 1

The efficacy tests were conducted by placing a CSR-wrapped tray (8.9cmx25.4cmx50.8cm; 3.5"x10"x20") consisting of representative medical devices and test lumens in a 20-liter aluminum chamber (11.2cmx30.5cmx55.9cm; 4.4"x12"x22"). A 2.5cm (one-inch) stainless steel wire inoculated with at least $1x10^6$ *Bacillus stearothermophilus* spores was placed in the center of each of the test lumens. The effects with and without temperature control of the diffusion path were investigated with both a TEFLON, poly(tetrafluoroethylene)lumen having an internal diameter of 1mm and a length of 700mm, and a stainless steel lumen having an internal diameter of 1mm, and a length of 500mm. All lumens were open at both ends. Each of the samples were subjected to a sterilization cycle in a 20 liter vacuum chamber, which was held at 40°C and 3 torr for 5 minutes. 1.44 ml of a 59% solution of hydrogen peroxide in water was injected at atmospheric pressure into the vaporizer which was held at 60°C. The 5 minute clock then started and the chamber was pumped down to 3 torr, which took less than one minute. In one case the diffusion path 24 had an initial temperature of 30°C for the first minute while the chamber was evacuated to 3 torr and was then heated to 50°C to release the condensed peroxide from the diffusion path into the chamber for the remainder of the cycle while pressure was maintained at 3 torr. In the other case, the diffusion path was held at 50°C throughout the cycle. By maintaining the diffusion path at 50°C, no or little peroxide was retained in the diffusion path. Sterilization effectiveness was measured by incubating the test samples in growth media at 55°C and checking for growth of the test organism. Table 1 shows the results of these tests.

TABLE 1

| Lumen Type | ID & Length | 50°C Diffusion Path Throughout Process | 30°C Diffusion Path For One Minute Then increased to 50°C |
|---|---|---|---|
| Teflon | 1 x 700 | 2/2 | 0/3 |
| Stainless Steel | 1 x 500 | 1/2 | 0/3 |

When the diffusion path temperature was maintained at high temperature throughout the process, all of the samples in the TEFLON lumen tested positive for bacteria growth, indicating failure of sterilization, and one of two samples in the stainless steel lumen tested positive. Under the same conditions, but with an initially lower temperature diffusion path which was heated starting one minute after the diffusion began, none of the samples tested positive. Condensing the peroxide in the diffusion path during the initial vaporization stage and then revaporizing the condensed peroxide from the diffusion path into the chamber greatly enhance the efficacy.

Additional efficiencies can be achieved by alternating cool and warm regions in the diffusion path 24 as primarily illustrated in FIG. 2. The temperature control elements 26, in simple form heating elements, are spaced apart from one another. Also, preferably, the diffusion path 24 is vertical in this respect. As the hydrogen peroxide solution vaporizes and passes through the diffusion path 24, it is thought that it may alternately condense and re-vaporize as it passes over the heated and unheated sections of the diffusion path 24. The diffusion path could alternatively comprise alternating heating and cooling elements.

The heater 32 within the chamber 12 acts similarly to the heating of the diffusion path 24. By controlling the heater 32 temperature, the peroxide can be first condensed on the heater 32 and then re-vaporized into the chamber 12 to concentrate the peroxide.

A preferred cycle would be a modification of a cycle described in the Wu et al. U.S. Patent No. 6,365,102. A series of pre-plasma energy additions with venting in-between dries moisture from the chamber 12. A vacuum is then drawn upon the chamber 12 and the hydrogen peroxide solution injected into the vaporizer 14. Alternatively, the peroxide solution can also be injected at atmospheric pressure. Some of the vaporizing solution condenses upon the cool diffusion path 24. After a time sufficient for most or all of the hydrogen peroxide solution to vaporize from the vaporizer 14, the diffusion path 24 is warmed by the temperature control elements 26 and the condensed hydrogen peroxide solution re-vaporizes. At about this time, the throttle valve 18 is closed and the pump 16 turned off to seal the chamber 12. Much of the water fraction of the hydrogen peroxide solution has thus been drawn out of the chamber 12 by the vacuum pump 16 and the remaining hydrogen peroxide solution which re-vaporizes from the diffusion path 24, or from the heater 32 in the chamber 12 if present, is of a higher hydrogen peroxide concentration than the starting solution. Preferably, a computer based control system (not shown) controls the functions of the process for ease and repeatability.

The hydrogen peroxide vapor thus produced contacts an article 34 or articles 34 in the chamber 12 and effects sterilization thereof. If those articles 34 have diffusion restricted areas, such as long, narrow lumens, it may be preferable to then vent the chamber 12 and allow clean sterile air therein to drive the hydrogen peroxide vapor deeper into the diffusion restricted areas. Then the chamber 12 is again subjected to vacuum and an additional injection of hydrogen peroxide, preferably with the heating sequence on the diffusion path, is repeated. After a time period sufficient to effect sterilization of the article 34, preferably with a six-log reduction in challenge organisms such as *Bacillus stearothermophilus,* a plasma is lit within the chamber 12, thereby enhancing the sterilization and breaking down the hydrogen peroxide into water and oxygen.

Plasma is generated by inducing a voltage between an anode 35 and a cathode which in this case is the interior wall 37 of the sterilization chamber 12. The anode 35 is a sheet of perforated metal spaced apart from and electrically insulated from the interior wall 37. US Patent Application Publication No. 20040262146, published December 30, 2004, describes in detail a particularly efficient method for generating such a plasma. Density of the plasma is defined in the space between the anode and cathode.

The orifice plate 20 can enhance the effect of concentrating the hydrogen peroxide during its vaporization. As described in the Lin et al. U.S. Patent No. 5, 851, 485, a controlled or slow pump-down of the chamber 12 initially draws off more water than hydrogen peroxide from solution as the water has a higher vapor pressure, thereby leaving a higher concentration hydrogen peroxide behind. Controlling the pump-down can be difficult as vacuum pumps generally do not throttle back well and throttle valves in such service are difficult to control and expensive. By placing the orifice plate 20 in the flow path to the pump 16, the amount of atmosphere from the chamber 12 exhausted by the pump 16 is limited, and by selecting a proper size orifice 36 in the plate 20 can be controlled to a rate which effectively concentrates hydrogen peroxide in the chamber 12.

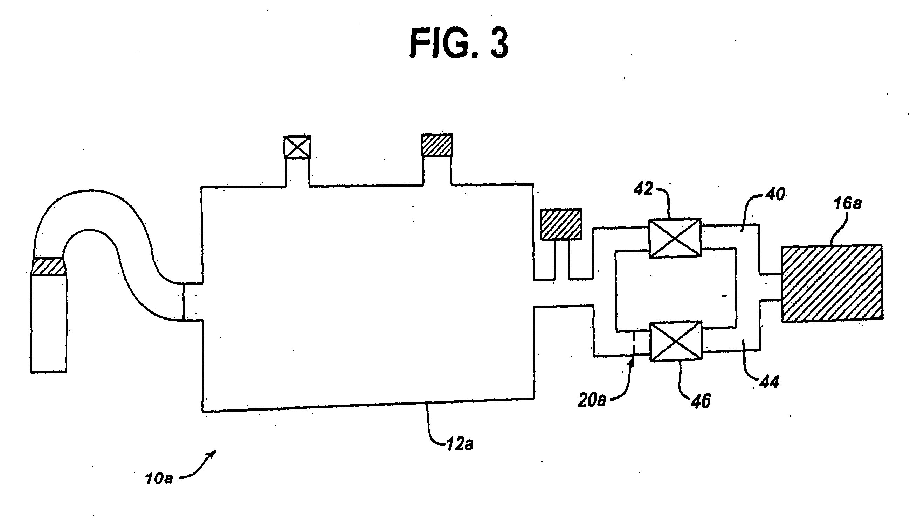

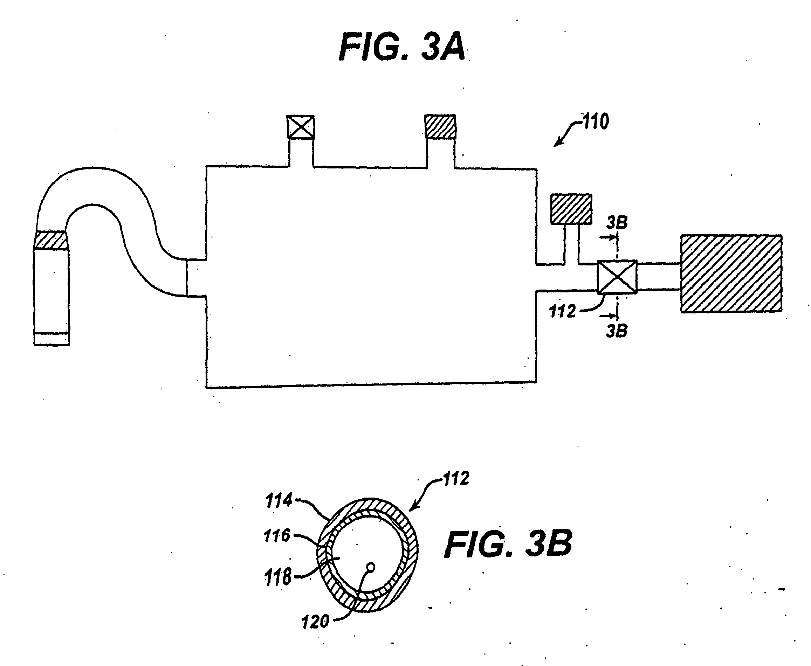

Turning also to FIG. 3, a system 10a, similar in most respects to the system 10 of FIGS. 1 and 2, with like part numbers denoted with an "a" appended thereto, also incorporates an orifice plate 20a. However, to allow a quick pump-down of the chamber 12a, yet retain the controlled pump-down benefits of the orifice plate 20a, it incorporates two path ways from the pump 16a to the chamber 12a. A first pathway 40 contains a throttle valve 42 and a second pathway 44 contains a throttle valve 46 and the orifice plate 20a. Thus, during initial pump-down the first throttle valve 42 is open leaving the pump 16a freely connected to the chamber 12a. As the chamber 12a approaches the vapor pressure of water, the first throttle valve 42 is closed thereby forcing the pump 16a to evacuate through the orifice plate 20a and thus draw out of the chamber 12a at a slower, controlled rate more conducive to preferentially drawing water out of the hydrogen peroxide solution and out of the chamber 12a.

Turning also to FIGS. 3A and 3B, a system 110 similar to that of FIG. 1 is shown. Here, rather than use two paths as in the system 10a of FIG. 3, a valve 112 comprises a valve body 114, a valve seat 116 and a valve element 118, such as a butterfly disc, plug or the like. An orifice 120 is provided through the valve element. Thus, when the valve 112 is open evacuation can occur quickly, and when the valve 112 is closed it can occur more slowly. Such a valve could also be employed between the vaporizer 14 and the chamber 12 to further control the preferential vaporization and removal of the water from the germicide solution.

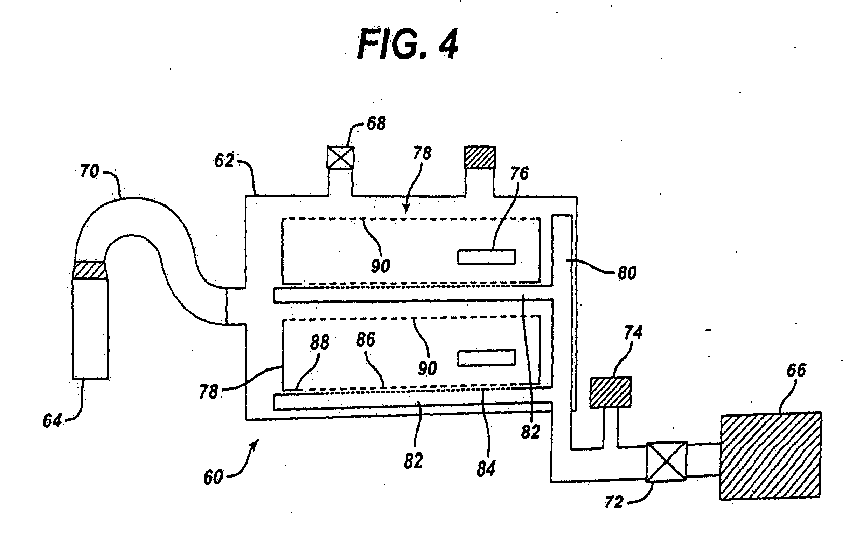

Turning now to FIG. 4, while highly concentration of the sterilizing vapor is helpful in achieving sterilization efficiency and efficacy, getting the vapor into contact with the items to be sterilized is also a concern. Typically, the low pressures (0.5 torr to 10.0 torr) inside of a chamber 12 promotes quick diffusion of the sterilant vapor to all areas therein.

FIG. 4 illustrates a sterilization system 60 comprising a chamber 62 having a vaporizer 64, vacuum pump 66 and vent 68 connected thereto. Preferably, an elongated, temperature controlled diffusion path 70 as previously described connects the vaporizer 64 to the chamber 62. A throttle valve 72 and pressure gauge 74 are provided at the pump 66.

Articles 76 to be sterilized are placed into trays or containers 78. Two types of packaging are commonly used in preparing articles 76 for sterilization. In one, the articles 76 are placed into a tray having a plurality of openings therein, and the tray is then wrapped with a material such as CSR wrap which passes sterilizing gases and blocks contaminating microorganisms. Such a tray is described in the Wu, U.S. Patent No. 6,379,631, An alternative package comprises a sealable container with several ports, preferably on top and bottom surfaces thereof, with each of the ports covered by a semi-permeable membrane which passes sterilizing gases and blocks admission of contaminating microorganisms. Such a container is described in Nichols U.S. Patent No. 4,704,254-. The first type of packaging is typically called a "tray" and the second a "container." However, the term "container" as used herein is meant to refer to any container, packaging or enclosure suitable for containing articles to be sterilized in a chemical vapor environment.

The pump 66 connects to the chamber 62 via an exhaust manifold 80. The manifold 80 comprises one or more shelves 82 for supporting and receiving one or more containers 78 and which connect fluidly through the throttle valve 72 to the pump 66. An opening, or preferably a plurality of openings 84 on the upper surfaces of the shelves 82 allow the pump 66 to draw atmosphere within the chamber 62 through the openings 84, through the manifold 80 and out through the pump 66.

The containers 78 preferably have openings 86 on a lower surface 88 thereon and additional openings 90 on at least one other surface. When the containers 78 are placed on the shelves 82 atmosphere being exhausted by the pump 66 is drawn in part through the openings 90 into the container 78, through the container into contact with the article or articles 76 therein and then out through the openings 86 into the manifold 80 through the openings 84 therein. When the atmosphere being so exhausted contains a sterilizing gas it enhances its penetration into the containers 78 and into contact with the articles 76 therein.

Sterilizing gases are so exhausted during the previously described cycle as the sterilant solution is vaporizing and immediately before the second admission of hydrogen peroxide. Such a cycle can also further provide a pump-down after some period of diffusion. After admitting the sterilant vapor the chamber 62 pressure rises slightly due to the presence of additional gas therein, typically from about 0.5 torr to about 10 torr. Higher pressures are as efficient with higher load and chamber temperatures.

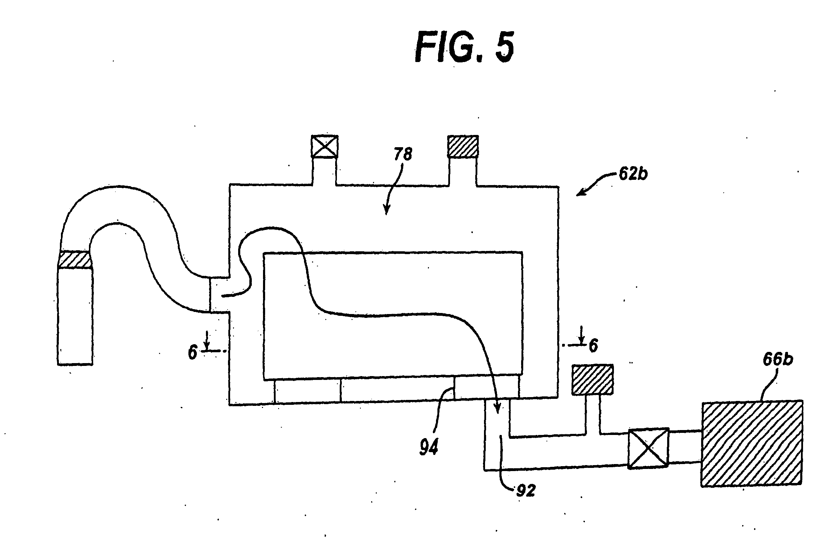

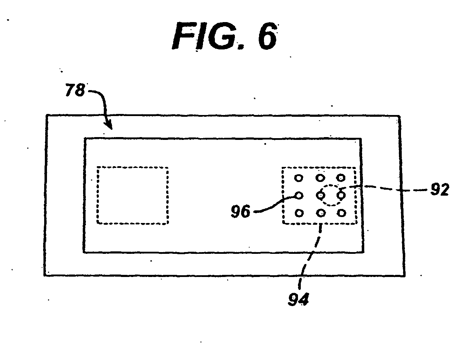

Turning also to FIGS. 5 and 6, an alternative design (in which like part numbers to those of the design of FIG. 4 are designated with a "b" appended thereto) replaces the manifold 80 of the design of FIG. 4 with a simple port 92. The port 92 is covered by a support 94 for the container 78, the support 94 having a plurality of openings 96 therethrough so that the chamber 62b is in fluid communication with the pump 66b through the container 78, the support 94 and the port 92. The support 94 can be removable.

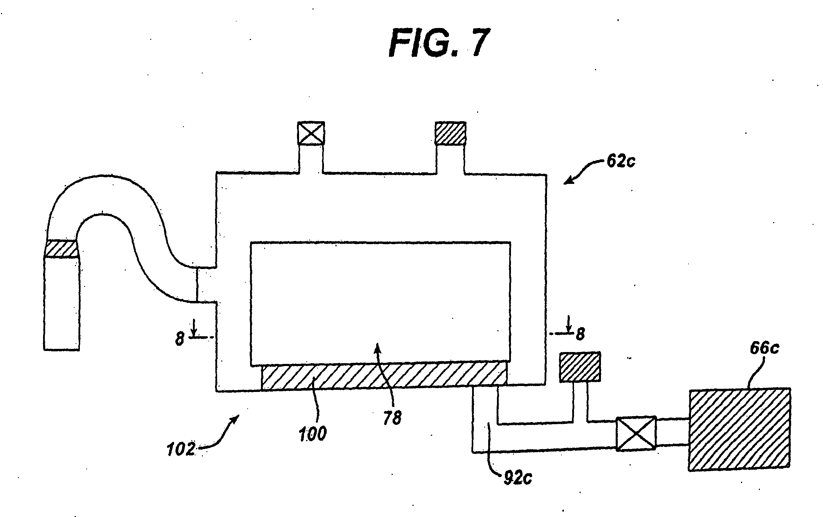

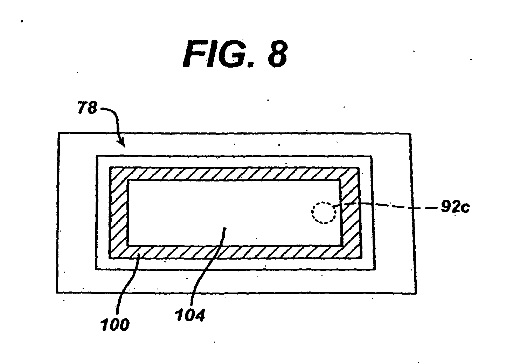

Turning also to FIGS. 7 and 8 (in which like part numbers to those of the designs of FIGS. 4 to 6 are designated with a "c" appended thereto) shows a support 100 resting on a surface 102 in the chamber 62c through which penetrates the port 92c. The support 100 surrounds the port 92c. Thus, most or all of the atmosphere being exhausted by the pump 66c passes through the container 78 into a space 104 formed between the container 78, the support 100 and the surface 102 and then onto the pump 66c through the port 92c.

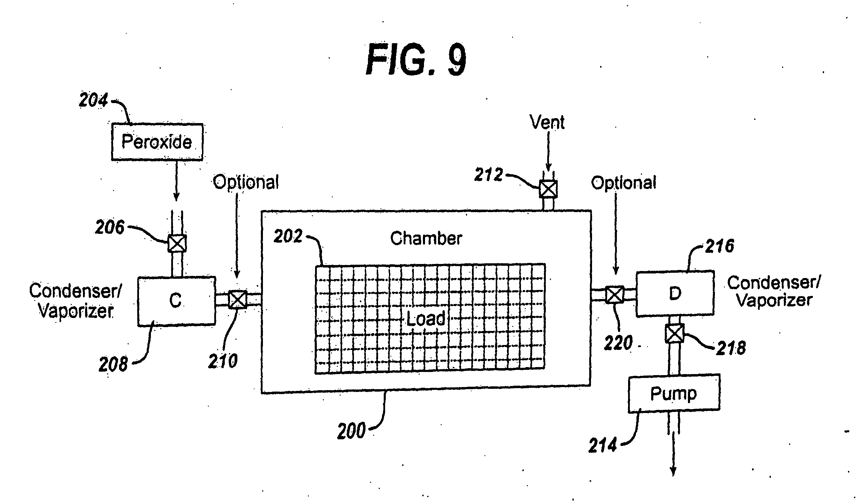

FIG. 9 discloses an alternative system in which, similar to the system of FIG. 1, a portion of the vaporized germicide solution can be condensed and the solvent, typically water, which has not condensed as quickly is removed from the atmosphere to further concentrate the germicide. The germicide is then revaporized to produce a more concentrated germicidal vapor for more efficient sterilization. The system comprises a sterilization chamber 200 containing a load 202 of items to be sterilized. A source 204 of liquid germicide solution provides the solution through a valve 206 to a first vaporizer/condenser 208 where it is vaporized and then supplied to the chamber 200. A valve 210 can be provided to isolate the vaporizer/condenser 208 from the chamber 200. The chamber 200 is also provided with a valved vent 212.

A vacuum pump 214 provides for lowering the chamber pressure as described in reference to the previous embodiments. Between the pump 214 and the chamber 200 a second vaporizer/condenser 216 is provided for condensing the vaporized solution. Preferably valves 218 and 220 isolate the second vaporizer/condenser 216 from the pump 214 and chamber 200 respectively.

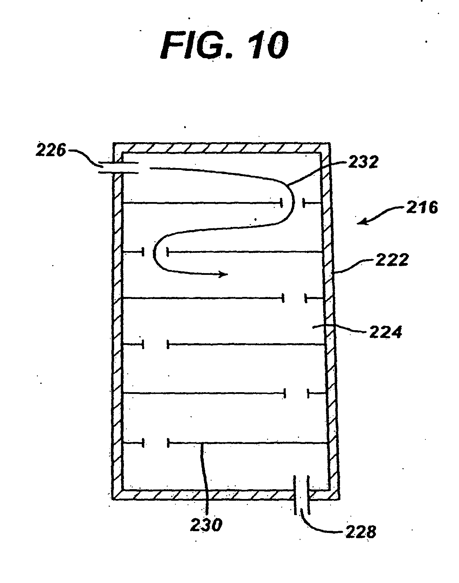

Turning also to FIG. 10 a simple version of the second vaporizer/condenser 216 preferably comprises walls 222 defining an enclosure 224 having an inlet 226 connected to the chamber 200 and an outlet 228 connected to the pump 214. A plurality of baffles 230 provides a torturous flow path 232 through the vaporizer/condenser 216. The walls 222, and potentially the baffles 230, are temperature controllable to enhance condensation of and re-vaporazation of the solution.

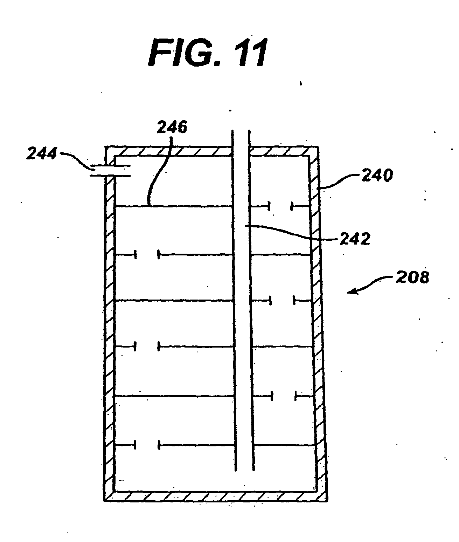

A similar structure with an inlet can be employed on the first vaporizer/condenser 208 as well. Turning also to FIG. 11, a simple version of the first condenser/vaporizer 208 is illustrated. It comprises an enclosure 240 having an inlet 242 connected to the source of solution 204 (not shown in FIG. 11) and an outlet 244 connected to the chamber 200 (not shown in FIG. 11). A plurality of baffles 246 provides a tortuous flow path through the first vaporizer/condenser 208. The enclosure 240 and potentially the baffles 246 are temperature controllable to enhance condensation and revaporization of the solution.

In a simple cycle, a liquid germicide solution, such as hydrogen peroxide and water is admitted into the first vaporizer/condenser 208 where it is vaporized and then flows into the chamber 200 which is at a low pressure, all as described in reference to previous embodiments herein. During vaporization and for sometime thereafter pump 214 continues to exhaust atmosphere from the chamber 200. By controlling temperature and pressure this preferentially vaporizes water from the solution over the hydrogen peroxide and the water vapor is extracted from the system via the pump 214 to concentrate the hydrogen peroxide solution during the vaporization phase. Additionally, hydrogen peroxide, having the lower vapor pressure, will tend to condense more quickly than the water vapor in the first vaporizer/condenser 208. As the pump 214 continues to exhaust atmosphere from the chamber 200 the vaporized hydrogen peroxide solution flows out of the chamber and into the second vaporizer/condenser 216 where a portion thereof will condense. Due to the preferential condensation of hydrogen peroxide over the water more of the water vapor will pass through the condenser 216 uncondensed and be exhausted via the pump 214 thus allowing further concentration of the hydrogen peroxide solution. At some point, the pump is turned off and the valve 218 closed. The condensed hydrogen peroxide within the vaporizer/condenser 216 is then re-vaporized preferably by heating the condenser 216. This hydrogen peroxide will have a higher concentration for more efficient sterilization of the load 202.

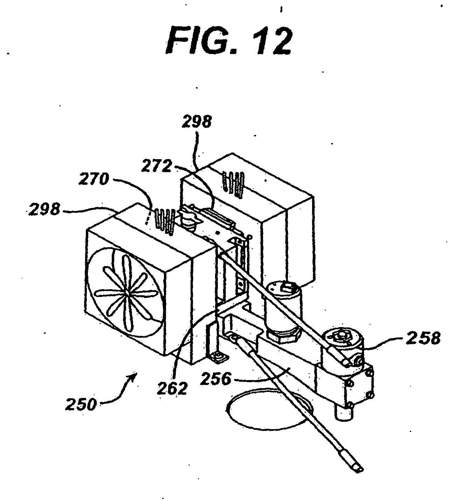

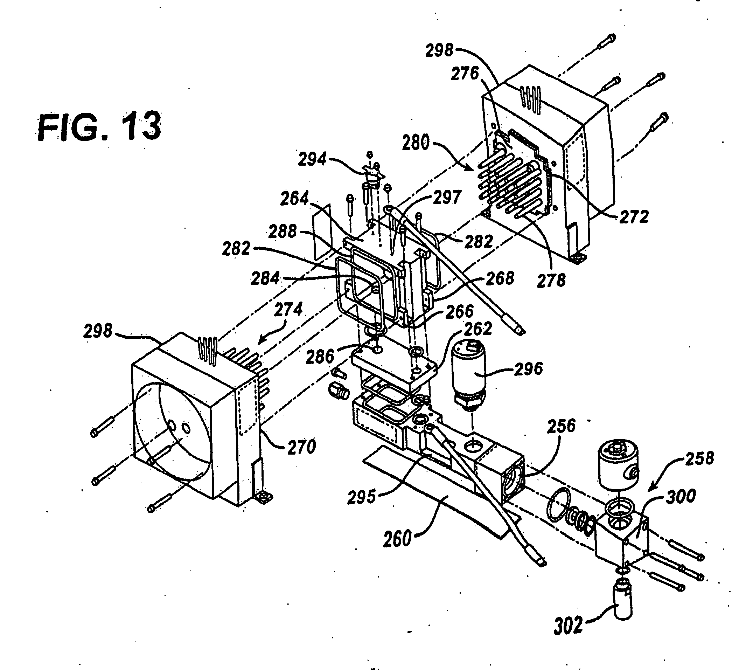

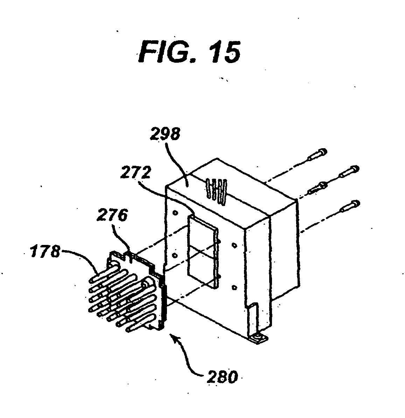

Turning also to FIGS. 12 through 15, a more elaborate condenser/vaporizer 250 is illustrated. In gross, it comprises an inlet manifold 252 which connects to the source of sterliant solution 204 and which provides initial vaporization, a condensing/revaporization section 254, an outlet manifold 256 and a control valve 258 via which the vaporizer/condenser 250 connects to the chamber 200. A resistance heater 260 affixes to the inlet manifold 252 and to the outlet manifold 256 to provide heat to assist in the initial vaporization within the inlet manifold 252 and to prevent condensation in the outlet manifold 256. Preferably, the inlet manifold 252 and outlet manifold 256 are formed of aluminum. Further, an insulator 262 is provided between the inlet manifold 252 and the vaporizer/revaporizer section 254.

The vaporizer/revaporizer section 254 comprises a housing 264, preferably formed of aluminum, open on a first side 266 and second side 268. A first thermo-electric device 270 and second thermo-electric device 272 affix to the first side 266 and second side 268, respectively. The thermoelectric devices 270 and 272 preferably operate under the Peltier effect, although other classes of thermoelectric devices could be substituted therefor. More conventional heat pumps, such as freon or ammonia based systems can also be employed with somewhat greater complexity.

A first rod assembly 274, comprising a plate 276 and a plurality of rods 278 extending normally therefrom affixes to the first thermo-electric device 270 with the rods 278 extending laterally into the housing 264. A second rod assembly 280 similarly attaches to the second thermo-electric device 272 with its rods 278 extending laterally into the housing 264 in facing relationship to the first rod assembly 274. The rod assemblies 274 and 280 are preferably formed of aluminum.

Preferably, the rods 278 extend almost to, without touching, the opposing plate 276. Also, the rods 278 from the two rod assemblies 274 and 280 lie in a generally parallel relationship with each other with a spacing therebetween designed to, along with the volume within the vaporizer/revaporizer section 254, provide a preferred flow rate of the vaporized sterliant therethrough to provide efficient condensation on to the rods 278. Preferably, a flow rate is in the range of 3cm/sec to 152cm/sec (0.1 ft/sec to 5 ft/sec), and more preferably a flow rate of 7.3cm/sec (0.24 ft/sec) is provided.

In a small condenser with a vapor path length of 7.6cm (3 inches), the residence time would be 1 second at a preferred velocity of 7.3cm/sec (0.24 ft/sec). This residence time would be sufficient for the vaporized sterilant to interact with the cooler condenser surfaces and to condense. For a typical injection volume of 2 ml of sterilant solution, the surface area of the condensing/revaporization section 254 would be about 90 square inches to permit mass transfer for condensation. High temperature at low pressure in the initial vaporizer (inlet manifold 252) maintains the water and hydrogen peroxide in the vapor phase for delivery to the condensing/revaporization section 254. For example, a vaporizer temperature of 70 degrees C or greater at a pressure of 125 torr or lower ensures that a 59 wt% solution of hydrogen peroxide and water will be in the vapor phase.

As vapor enters the condensing/revaporization section 254, which has a lower temperature, the hydrogen peroxide condenses on the cooler surface forming a concentrated solution. The temperature and pressure therein determine the concentration of the condensed solution. For example, at 50 degrees C and 13 torr in the condensing/revaporization section 254, the condensed hydrogen peroxide concentration would be 94 wt%. At 30 degrees C and 3.8 torr, the condensed hydrogen peroxide concentration also would be 94 wt%. As the pressure in the condensing/revaporization section 254 is lowered, the temperature must also be lowered to maintain the same concentration of solution.

The orifice 308 offers the advantage of a more concentrated solution by restricting the flow from the condensing/revaporization section 254 to provide a more controlled vaporization. Variations in pressure in the condensing/revaporization section 254 and in the vaporizer due to vacuum pump pressure fluctuations are dampened out by the orifice 308 to prevent surges of water vapor from carrying hydrogen peroxide droplets from the condensing/revaporization section 254. Another advantage of flow restriction by the orifice 308 is achieving a low pressure (less than 1 torr) in the sterilization chamber 200 to improve the diffusion coefficient in lumens while maintaining a greater pressure in the vaporizer/condenser 250 to operate at a greater temperature in the condensing/revaporization section 254. Without an orifice 308, sterilization chamber 200 and vaporizer/condenser 250 pressures must both be reduced to the same low pressure together, and the condenser must be operated at a very low temperature to maintain equilibrium of the solution. A lower condenser temperature is more difficult to control and may produce ice or condensate, which requires a more expensive design to protect electrical equipment.

An O-ring 282 seals the plates 276 on the thermo-electric devices 270 and 272 against the housing 264. An aperture 284 through the housing 264 aligns with an aperture 286 through the insulator 262 to place a chamber 288 defined by the housing 264 into fluid communication with the inlet manifold 252. An outlet passage 290 in the housing 264 connects to an upper portion of the chamber 288 and to a second aperture 292 through the insulator 262 which in turn aligns with the outlet manifold 256 to place the chamber 288 in fluid communication with the outlet manifold 256. A safety thermostat 294 atop the housing 264 is wired outside of the control system to shut down heating of the vaporizer/condenser 250 above a predetermined temperature. Temperature sensors 295 and 297 measure temperature in the inlet manifold 252 and condensing/revaporization section 254 respectively. A pressure sensor 296 interfaces with the outlet manifold 256. Heat sinks 298 having fan housings attach to each of the thermo-electric devices 270 and 272.

The outlet manifold connects to a valve manifold 300 which provides three possible flow paths between the vaporizer/condenser 250 outlet manifold 256 and a valve manifold outlet 302 from the valve manifold 300. The valve manifold outlet 302 communicates with the main chamber 200. A main flow passage 304 is controlled by a valve 306 which can open to allow flow through the main passage 304 to the valve manifold outlet 302 or close to block such flow. The second passage is through an orifice 308 in an orifice plate 310 which provides a flow restriction to enhance the ability to preferentially draw water vapor from the vaporizer/condenser 250. A third potential passage is through a rupture disk 312 which is designed to rupture in case of a catastrophic overpressure within the housing chamber 288, such as in the unlikely event that an oxidizable sterliant such as hydrogen peroxide combusts therein. The orifice 308 could be moved to a position within the shut-off valve 306, similar to that described in reference to the valve element 118 in FIGS. 3A and 3B.

In operation, the main chamber is first evacuated to a low pressure sufficient to induce vaporization, such as 0.4 torr and the valve 306 is closed placing the vaporizer/condenser 250 into fluid communication with the chamber 200 solely through the orifice 308. The inlet manifold 252 is heated with the heater 260 and a quantity of sterliant solution such as a 59% hydrogen peroxide/water solution is injected into the inlet manifold 252 where it vaporizes and diffuses into the housing 264 through the apertures 286 and 284. The thermo-electric devices 270 and 272 at this time are drawing energy out of the rods 278 and dissipating it through the heat sinks 298 thus allowing the vaporized sterliant to recondense on the rods 278.

The temperature of the inlet manifold 252 can be controlled to slowly vaporize the sterilant thus allowing the water to more quickly vaporize and flow through the vaporizer 250 and out through the orifice 308 to concentrate the remaining sterilant. The condenser/revaporization section 254 quite effectively concentrates the sterilant such that to speed up the process a fast vaporization in the inlet manifold can be employed while still achieving a high degree of concentration.

The condensate on the rods 278 tends to be more highly concentrated in the sterilant. After a time, when the initial charge of sterilant solution has been vaporized and a portion thereof condensed on to the rods 278, the thermo-electric devices 270 and 272 are reversed to apply heat to the rods 278 and revaporize the sterilant. At this time, the heat sink 298 will still contain heat which had been extracted during the prior step and that heat can be used by the thermo-electric devices 270 and 272 to very efficiently heat the rods 278 and revaporize the sterilant. This added efficiency improves the energy efficiently of the device and allows a smaller and more compact vaporize condenser 250 to provide adequate heating and cooling. After the sterilant has been revaporized, the valve 306 is opened to allow efficient diffusion of the sterilant vapor into the main chamber 200.

If a second vaporizer/condenser 216 is employed, its structure preferably mimics that of the vaporizer/condenser 250 without the inlet manifold 252.

In such a system, after initial diffusion into the main chamber 200, rods within the second condenser 216 would be chilled and the pump 214 turned on to preferably extract water vapor from the condensing sterilant. After a period of time when sterilant has condensed, the rods would be heated to revaporize the sterilant and the pump 214 turned off. This revaporized sterilant would have somewhat higher concentration and would then re-diffuse into the chamber 200 to further enhance the sterilization process.

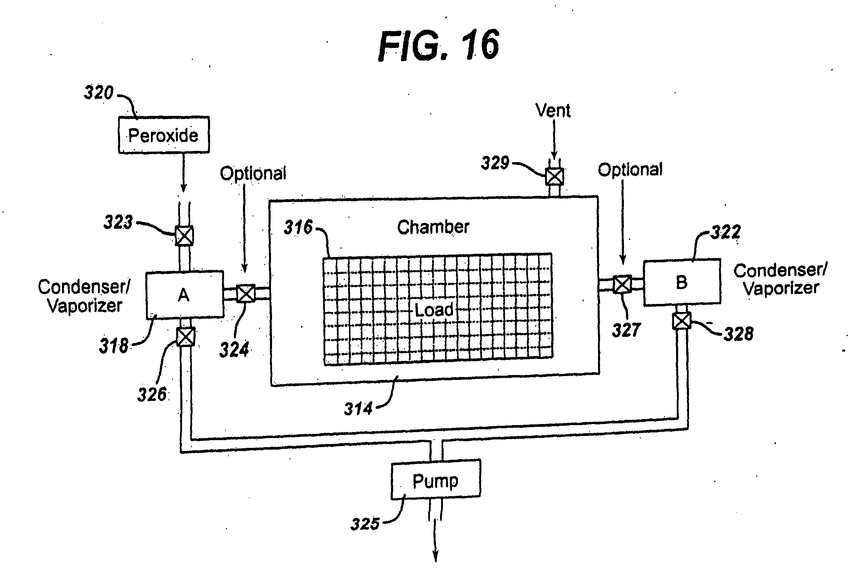

Other system arrangements are possible. FIG. 16 illustrates an alternative embodiment which can enhance efficiency in conserving and concentrating the germicide solution. In this system, a chamber 314 containing a load 316 has a first condenser/vaporizer 318 connected to a source 320 of germicide solution and a second condenser/vaporizer 322. The first condenser vaporizer 318 is isolated from the source 320 by a valve 323 and from the chamber 314 by a valve 324. It also connects to an exhaust pump 325 and is isolated therefrom via a valve 326. The second condenser vaporizer 322 is isolated from the chamber 314 by a valve 327 and connects to the pump 325 and is isolated therefrom via a valve 328. A vent 329 is also provided.

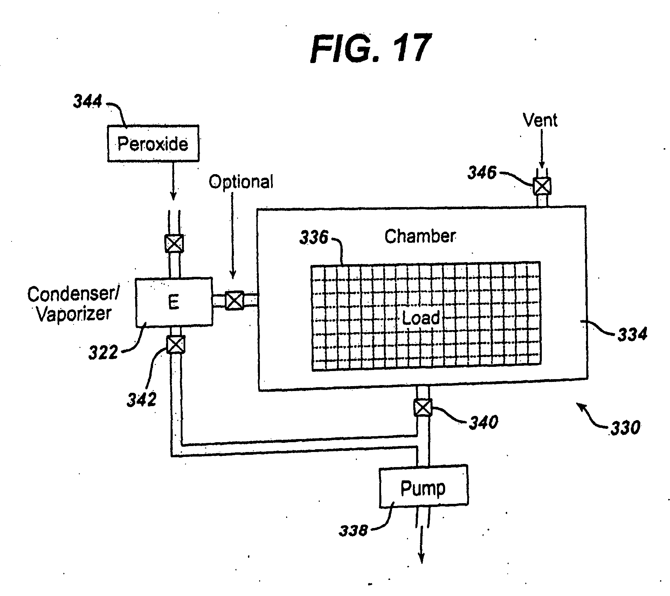

FIG. 17 illustrates a similar system 330 employing a single condenser/vaporizer 332 (of structure similar to the condenser/vaporizer 250 with an additional outlet) connected to a sterilization chamber 334 adapted to receive a load 336 of instruments to be sterilized. A vacuum pump 338 connects to the chamber 334 via a valve 340 and to the condenser/vaporizer 332 via a valve 342. A three-way valve may substitute for valves 340 and 342. A source of germicidal solution 344 connects to the condenser/vaporizer 332 and the chamber 334 has a vent 346. During initial vaporization and concentration of germicide from the source 344, valve 342 is closed. After the vapor is diffused into the chamber 334, valve 340 can be closed and the pump 338 used to draw vapor out of the chamber through the condenser/vaporizer 332 in its condensing mode to further concentrate the germicide. The concentrated germicide is then revaporized and diffused back into the chamber 334.

The second condenser/vaporizer 216 of FIG. 9 can be used to maximize germicide utilization when running a sterilization process with two full cycles of vacuum, inject, diffuse and vent. Prior to venting during the first cycle, the pump 214 is run with the condenser/vaporizer 216 being chilled to condense the germicide therein. The valves 220 and 218 are closed during the venting process. During the subsequent pump down, the condenser/vaporizer is kept chilled to keep the germicide from unduly vaporizing and being carried out of the system.

The systems of FIGS. 16 and 17 allow even more of the germicide to be retained between cycles in a two cycle process. Prior to venting in the first cycle germicide is condensed into the condenser/vaporizer 332. However, during the subsequent pump down it can be isolated from the pump via the valve 342 thus minimizing the tendency of the pump 338 to pump the saved germicide out of the system during pump down.

In each of this type of system the steps of condensing and concentrating the vaporized germicide and then revaporizing it can be repeated as needed to further concentrate the germicide.

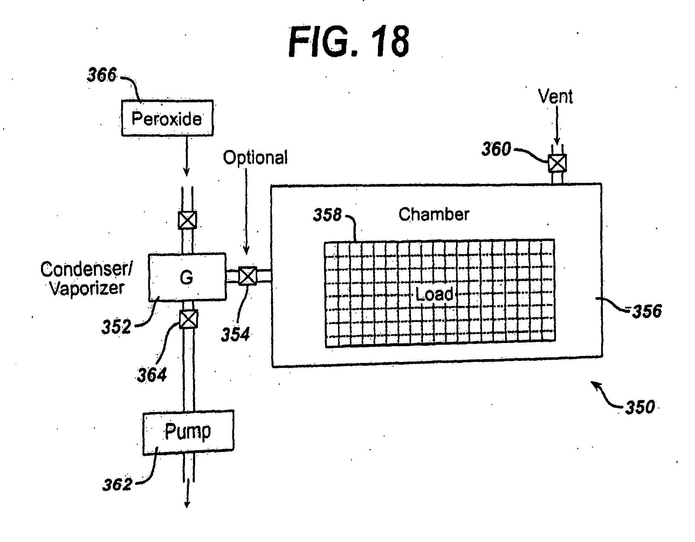

FIG. 18 illustrates a system 350 plumbed in an alternative fashion. In this system 350 a condenser/vaporizer 352 connects through a valve 354 to a sterilization chamber 356 adapted to receive a load 358 and having a vent 360. A vacuum pump 362 connects to the condenser/vaporizer 352 through a valve 364, but has no separate connection to the chamber 356. A source 366 of germicide connects to the condenser/vaporizer 352.

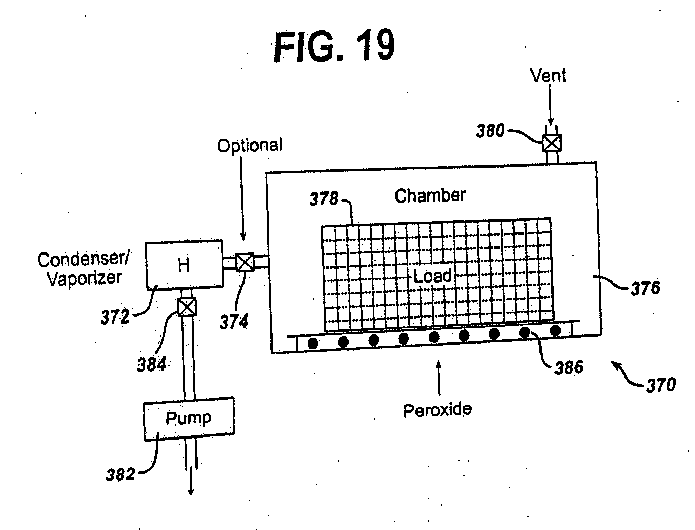

FIG. 19 illustrates a system 370 plumbed as in FIG. 17, having a condenser/vaporizer 372 which connects through a valve 374 to a sterilization chamber 376 adapted to receive a load 378 and having a vent 380. A vacuum pump 382 connects to the condenser/vaporizer 372 through a valve 384, but has no separate connection to the chamber 356. Rather than an inlet for germicide through the condenser/vaporizer 382, a source 386 of germicide solution is provided within the chamber 376. The source can be simple such as a well containing a quantity of liquid germicide solution. Preferably, it is covered with a semi-permeable membrane or filter so that liquid germicide can not be accidentally spilled therefrom yet as the germicide vaporizes under low chamber pressures the vapors thus generated can pass through the membrane into the chamber. In both systems the condenser/vaporizer 352 or 372 concentrates the germicide via condensation and revaporization of germicide vapor as described above.

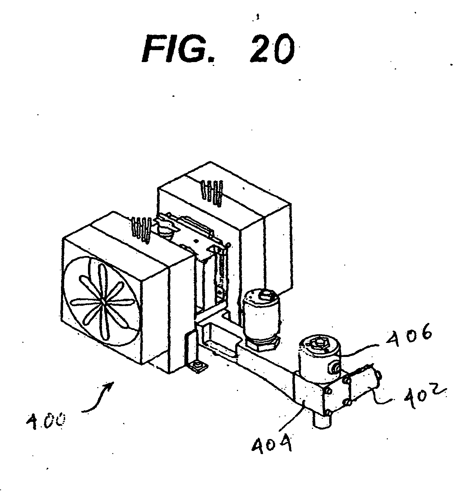

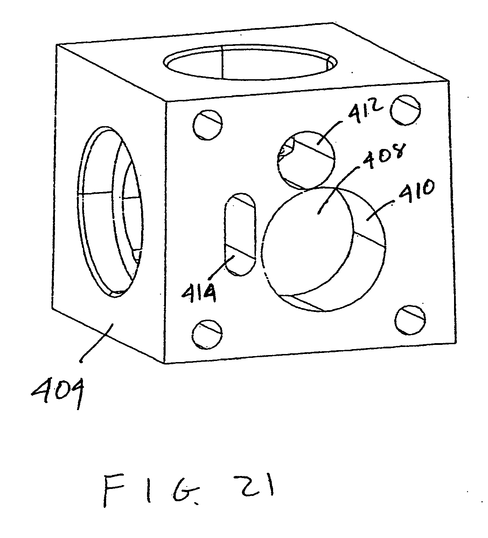

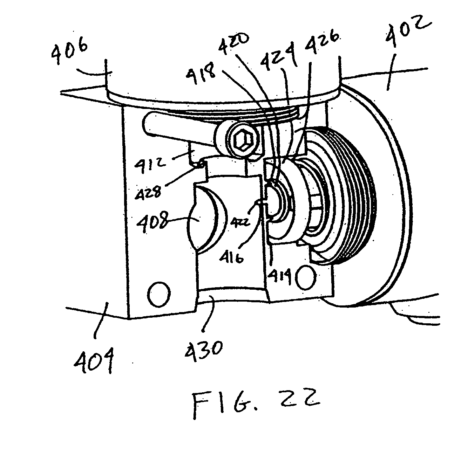

FIG. 20 illustrates a further embodiment of an inlet condenser/vaporizer 400. It is similar in most respects to that illustrated in FIG. 12. However, as shown primarily in FIGS. 21 and 22, it features an orifice control valve 402. A valve block 404 receives an outlet control valve 406, a rupture disk 408 and the orifice control valve 404.

FIG. 21 shows the valve block 404 in isolation and illustrates three manifold passages which connect the valve block 404 to the rest of the condenser/vaporizer 400: a large pressure relief manifold passage 410 which leads to the rupture disk 408, a smaller upper manifold passage 412 which leads to the outlet control valve 406 and a smaller lateral manifold passage 414 which leads to an orifice 416 and the orifice control valve 402.

FIG. 22 best illustrates the orifice control valve 402. A valve seat 418 on the valve block 404 surrounds the orifice 416. A valve member 420 on the orifice control valve 402 can extend toward to valve seat 418 to seal against it and block fluid communication through the orifice 416. A cleaning pin 422 penetrates the orifice 416 when the orifice control valve 402 is closed to clean the orifice 416 and keep it clear of foreign matter. An annular guide 424 connected to the valve member 420 slides within a bore 426 within the valve block 404 to properly align the cleaning pin 422 with the orifice 416. This view also illustrates a valve seat 428 for the outlet control valve 406 and a valve block outlet passage 430 which leads to the sterilization chamber (not shown in FIGS. 20 to 22).

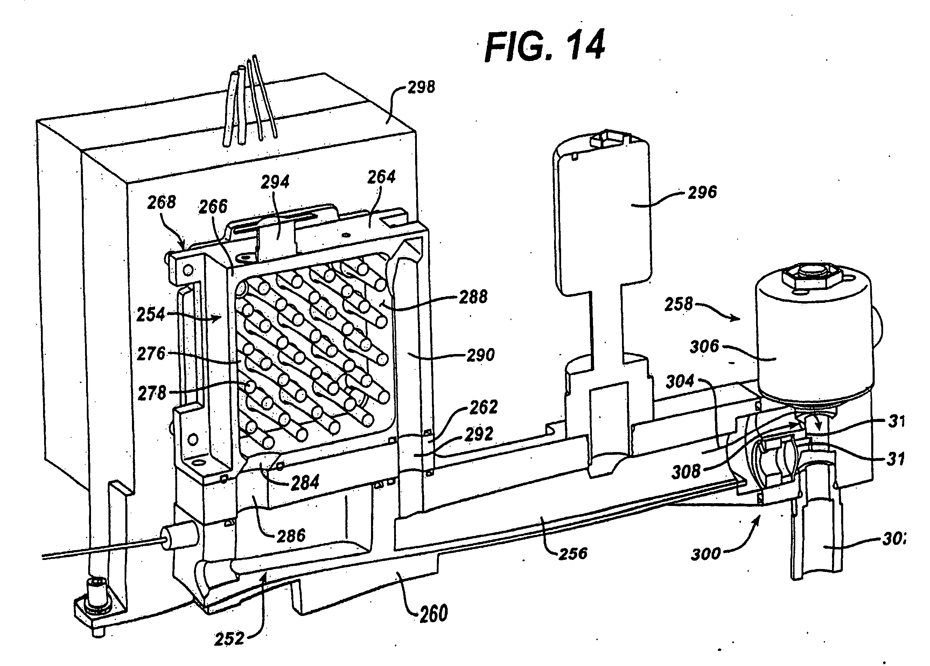

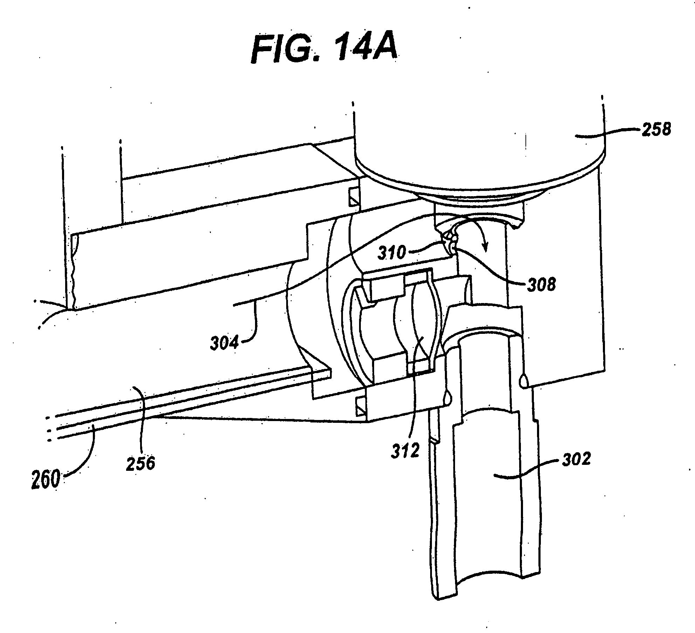

Operation of a sterilization cycle proceeds nearly the same as afore-described regarding the system shown in FIGS. 12 to 15. However, after the initial vaporization of the sterilant in the inlet manifold 252 (see FIG. 14) the orifice control valve 402 is closed thereby isolating the condenser/vaporizer 400 from the sterilization chamber (not shown in FIGS. 20 to 22). This condition can be monitored most easily be monitoring the pressure within the vaporizer/condenser 400 and assuming that when a particular pressure has been reached that essentially all of the sterilant has been vaporized. Pressure in the sterilization chamber is then reduced, preferably to approximately 0.5 Torr. The outlet control valve 406 is then opened and the rods 278 (see FIG. 14) are heated to vaporize condensed sterilant and pass it through the outlet control valve 406 and outlet passage 430 to the sterilization chamber.

By lowering the pressure in the sterilization chamber prior to admitting the bulk of the sterilant it has been found that overall cycle times may be reduced. Closing the orifice control valve 402 and reducing pressure in the sterilization chamber takes additional time. However, the lower pressure provides a more favorable condition for diffusion of the sterilant into diffusion restricted areas, such as lumens, of instruments to be sterilized. It has been found that the time saved through the increased diffusion efficiency can more than offset the time lost in lowering the pressure in the sterilization chamber. Sterilization cycle speed is an important factor for sterilizer users.

Water vapor in the sterilization chamber can affect the time required to lower the pressure therein. Such water vapor typically arises from a load of instruments that have not been properly dried. If undue time is required to remove the water vapor it can be indicated to the user so that they can be reminded to be more vigilant in drying the load for future cycles. There may exists loads of water vapor for which it may take too long to withdraw or to withdraw effectively. In such case the cycle should be cancelled and the user informed as to why.

Table 2 shows control points for three different cycles - a flash or very quick cycle having no lumens, a short cycle having only lumens which present a mild challenge and a long cycle for sterilizing devices with more challenging long and narrow lumens. During an initial pump-down to remove air from the sterilization chamber and vaporizer/condenser 400 the outlet control valve 406 is left open. As the pressure reaches P1 the outlet control valve 406 is closed but the orifice control valve 402 is left open; this starts the vaporization and concentration of the sterilant. Upon reaching pressure P2 within the vaporizer/condenser 400 the pressure Pc within the chamber is checked. If it is above the value listed in Table 2 then the orifice control valve 402 is closed and pump-down continues until Pc is reached and then the outlet control valve 406 is opened to transfer the sterilant into the sterilization chamber. Otherwise, the outlet control valve 406 is opened right away. If the chamber pressure exceeds Pc-cancel at the time that the vaporizer/condenser pressure reaches P2 it is assumed that the sterilization chamber contains too much water and the cycle is cancelled.

Table 2

| Examples of temperature and pressure set points | | | |
|---|---|---|---|
| | Flash | Short | Long |
| Load condition | Surface | 1mmx150mm SS 1mmx350mm Plastic | 1mmx500mm SS 1mmx1000mm Plastic |
| Vaporizer temperature | 70°C | 70°C | 70°C |
| Condenser temperature | 58°C | 52°C | 43°C |
| P1 Vaporizer/condenser pressure to remove air | 140 torr | 140 torr | 140 torr |
| P2 Vaporizer/condenser pressure to concentrate sterilant | 22 torr | 16 torr | 10 torr |
| Pc Chamber pressure to select transfer, additional vacuum or cancellation | 1.5 torr | 0.6 torr | 0.3 torr |
| Pc-cancel Chamber pressure to cancel cycle | 8 torr | 6 torr | 4 torr |
| Condenser temperature to transfer concentrated sterilant | 68°C | 68°C | 68°C |

The vapor concentration of the process can also be measured in mg/L. It is preferably 6 mg/L to 25 mg/L or even higher, with values of about 20 to 25 mg/L being most preferred.

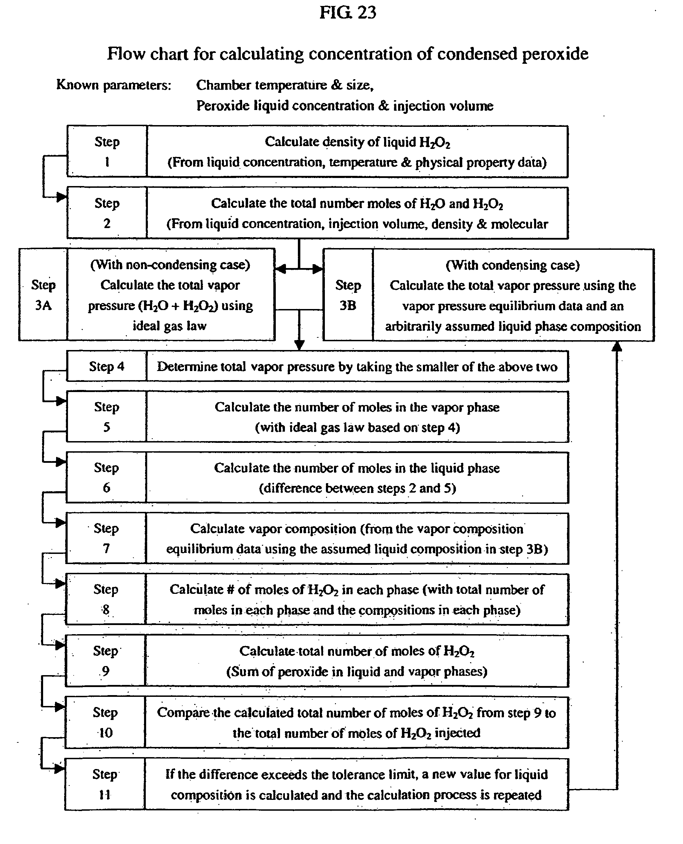

At these concentrations a portion of the injected hydrogen peroxide will condense upon the items to be sterilized. The subsequent pump down of the chamber re-vaporizes this condensed hydrogen peroxide thus further enhancing the concentration of hydrogen peroxide by weight percent. Figure 23 provides a flow chart for calculating the concentration of the condensed hydrogen peroxide and relies upon the following equations 1 to 10.

$$(1) \quad p = a + bw + cw^2 + dw^3 \quad \text{(where p is density \& w is wt\% } H_2O_2\text{)}$$

$$(2) \quad a = J_a + K_a t + L_a t^2 + M_a t^3$$

$$(3) \quad b = J_b + K_b t + L_b t^2 + M_b t^3 \text{ (where t is solution temperature in degree Celsius)}$$

$$(4) \quad c = J_c + K_c t + L_c t^2 + M_c t^3$$

$$(5) \quad d = J_d + K_d t + L_d t^2 + M_d t^3$$

The coefficients for equations 1 to 5 are shown in Table 3.

Table 3

|   | J | K*10³ | L*10⁵ | M*10⁷ |
|---|---|---|---|---|
| a | 0.99957 | 0.07603 | -0.8603 | 0.51204 |
| b | 0.39763 | -2.8732 | 3.2488 | -1.6363 |
| c | 0.02206 | 3.5357 | -6.0947 | 3.6165 |
| d | 0.05187 | -1.9414 | 3.9061 | -2.5500 |

$$(6) \quad \log p_{ho} \text{ (torr)} = 44.5760 - 4025.3 \text{ (1/T)} - 12.996 \text{ (log(T))} + 0.0046055 \text{ (T)}$$

(where $p_{ho}$ is vapor pressure of $H_2O_2$ in torr & T is temperature in degree Kelvin)

$$(7) \quad \log p_{wo} \text{ (torr)} = 30.75050 + 3120.5 \text{ (1/T)} + 7.921 \text{ (log(T))} + 0.0023172 \text{ (T)}$$

(where $p_{wo}$ is vapor pressure of $H_2O$ in torr & T is temperature in degree Kelvin)

$$(8) \quad P = p_{wo}x_w r_w + p_{ho}(1-x_w) r_h$$

(where

P is the total vapor pressure in torr
$X_w$ is the wt% of water concentration
$r_w$ is the activity coefficient of water
$r_h$ is the activity coefficient of hydrogen peroxide)

$$(9) \quad r_w = \exp(((1-x_w)^2)/RT)(B_0 + B_1(1-4x_w) + B_2(1-2x_w)(1-6x_w)))$$

(Where

R is the universal gas constant [62.36 torr*liter/mole*°K]
$B_0 = -752 + 0.97t = -1017 + 0.97T$ (where t is °C and T is °K)
$B_1 = 85$
$B_2 = 13$)

$$(10) \quad r_h = \exp(((x_w)^2)/RT)(B_0 + B_1(3-4x_w) + B_2(1-2x_w)(5-6x_w)))$$

In addition to the effectiveness of such systems in killing infectious microorganisms they are also effective in inactivating prions. Experiments were conducted comparing known methodologies to the highly concentrated hydrogen peroxide vapor of the present disclosure.

Steel Wire Preparation

Soft stainless steel wire (1.4301; diameter, 0.25 mm; Forestadent, Pforzhein, Germany) was placed in the Sterrad 100S GMP sterilizer, a hydrogen peroxide gas plasma sterilizer. The steel wire was then aseptically cut into fragments of 30 mm in length.

The wires were contaminated by immersion into freshly, prepared 10% brain homogenates from hamsters with scrapie in phosphate-buffered saline (PBS) for 16 hours at room temperature; spiked wires were then air dried for at least 1 hour before further treatment.

Cleaning was performed in a custom-made test washing machines, a closed two-container system. The lower container served as an open reservoir; a working volume of 10 L was pumped into the upper container and then drawn out freely back into the lower container through an opening on the bottom of the upper container. A circular rack with wires fixed in the edge was placed within the upper container and the lid of the upper container was tightly closed during the washing process.

After each treatment, wires were rinsed once with 1 x PBS followed by washing three times with double distilled water.

Wire Implantation

The processed wired were then implanted into hamster thalami with the assistance of a stereotaxic apparatus for small animals (coordinates: bregma, -2.0 mm; mediolateral, 2.0 mm; and dorsoventral, 6.0 mm). The same location was chosen for the intracerebral injection of scrapie brain homogenates into the hamster brain by a syringe and for the controls.

Animals were deeply anesthetized with 10% ketamine (SANOfi-CEVA GmbH, Dusseldorf, Germany) during the operation.

Animal Surveillance

Hamsters implanted with the wires were kept three to four per cage in a biological safety level-3 facility, with free access to standard diet and water. Test animals were initially observed twice a week, and daily after 60 days postimplantation. Hamsters with definite signs of scrapie were designated as terminally sick and euthanized.

Treatment Regiments Compared

Samples of the prion prepared wires were processed with the following treatments to assess their effectiveness in inactivating the prions on the wires. Twelve animals were used for each treatment, and five animals were used as control.:

  1. Neodisher V 4009/1; 1%; 55°C for 10 minutes (control)
  2. Neodisher V 4009/1; 1%; 55°C for 10 minutes and exposure to STERRAD NX - Advanced Cycle (38 min)
  3. Neodisher V 4009/1; 1%; 55°C for 10 minutes and exposure to STERRAD 100 S - Long Cycle
  4. Alka One; 1%; 55°C for 10 minutes (control)
  5. Alka One; 1%; 55°C for 10 minutes and exposure to STERRAD NX - Advanced Cycle (38 min)
  6. Alka One; 1%; 55°C for 10 minutes and exposure to STERRAD 100 S - Long Cycle
  7. Exposure to STERRAD NX - Advanced Cycle (38 min) only; no washing
  8. Exposure to STERRAD NX - Double Advanced Cycle (38 min) only; no washing
  9. Exposure to STERRAD 100 S - Long Cycle only; no washing
  10. Exposure to STERRAD 100 S - Double Long Cycle only; no washing

Neodisher V 4009/1 is an alkaline cleaning solution available from Chemische Fabrik Dr. Weigert GmbH & Co, Hamburg, Germany. Alka One is an alkaline cleaning solution available from Borer Chemie AG, Zuchwil, Switzerland. The STERRAD 100 S is available from Advanced Sterilization Products division of Ethicon, Inc. (ASP), Irvine, CA. Its long cycle comprises a 15-minute pre-exposure plasma with about 9.5 mWatts/cc power density and two 100S half cycles and its double long cycle comprises two long cycles. Each 100S half cycle comprises a 6-minute injection of 59% peroxide, a 10-minute diffusion, and a 2-minute plasma with about 9.5 mwatts/cc plasma power density. The chamber temperature is maintained around 45°C throughout the process.

The STERRAD NX is also available from ASP. Its advanced cycle comprises two NX half cycles and its double advanced cycle comprises two advanced cycles. Each NX half cycle comprises a concentration process, which concentrates the injected 59% peroxide to about 90%, a 7-minute transfer stage to transfer the concentrated into sterilizer, a 30-second diffusion, and a 4-minute plasma with about 50 mWatts/cc plasma power density. The chamber is maintained around 50°C throughout the process. Due to the higher chamber temperature, higher peroxide liquid concentration, higher plasma power density, and longer plasma time, the load in the STERRAD NX Sterilizer is actually exposed to more effective and energetic sterilization environment than the STERRAD 100S Sterilizer. The results of these trials are shown in Table 4 which shows the lifespan in days of the hamsters (as of the compilation of the data) after implementation with the treated wires. Figures in bold indicate a deceased animal and figures indicated with an asterisk indicate an animal deceased due to conflict with another animal.

Table 4

Comparison of Treatments to Inactivate Prions

| | (1) 1% V4009/1 55°C 10min | (2) AlkaOne 55°C 10min | (3)No wash NX 38min double | (4) No wash NX 38min | (5) 1% V4009/1 55°C 10min/NX 38min | (6) AlkaOne 55°C 10min/NX 38min |
|---|---|---|---|---|---|---|
| Implantation | 9/21/2005 | 9/22/2005 | 10/6/2005 | 10/7/2005 | 10/11/2005 | 10/13/2005 |
| Hamster 1 | 168 | 167 | 153 | 152 | 148 | 146 |
| Hamster 2 | 168 | 167 | 153 | 152 | 148 | 146 |
| Hamster 3 | 168 | 134* | 153 | 152 | 148 | 146 |
| Hamster 4 | 168 | 167 | 153 | 152 | 148 | 146 |
| Hamster 5 | 168 | 167 | 153 | 152 | 148 | 146 |
| Hamster 6 | 99* | 167 | 153 | 115* | 148 | 146 |
| Hamster 7 | 168 | 167 | 153 | 152 | 85* | 146 |
| Hamster 8 | 168 | 167 | 153 | 130* | 148 | 146 |
| Hamster 9 | 168 | 167 | 153 | 152 | 148 | 146 |
| Hamster 10 | 168 | 167 | 153 | 152 | 148 | 146 |
| Hamster 11 | 168 | 167 | 90* | 152 | 148 | 146 |
| Hamster 12 | 168 | 167 | 153 | 152 | 85* | 146 |

| | (7) AlkaOne 55°C 10min/100S long | (8) 1% V4009/1 55°C 10min/100S long | (9) No wash 100S long double | (10) No wash 100S long | Positive control wires |
|---|---|---|---|---|---|
| Implantation | 11/8/2005 | 11/8/2005 | 11/9/2005 | 11/9/2005 | 7/6/2005 |
| Hamster 1 | 120 | 120 | 107 | 93 | 75 |
| Hamster 2 | 120 | 120 | 103 | 103 | 85 |
| Hamster 3 | 120 | 120 | 97 | 107 | 85 |
| Hamster 4 | 120 | 120 | 93 | 93 | 85 |
| Hamster 5 | 120 | 120 | 119 | 97 | 85 |
| Hamster 6 | 120 | 120 | 107 | 103 | |
| Hamster 7 | 120 | 120 | 103 | 93 | |
| Hamster 8 | 120 | 120 | 107 | 93 | |
| Hamster 9 | 120 | 120 | 107 | 107 | |
| Hamster 10 | 120 | 120 | 93 | 107 | |
| Hamster 11 | 120 | 120 | 107 | 93 | |
| Hamster 12 | 120 | 120 | 103 | 93 | |

The results show the high concentration hydrogen peroxide vapor treatment of the STERRAD NX to be as effective as alkaline wash treatment in inactivating prions.

**Claims**

1. A method of inactivating prions comprising the steps of:

placing an article having a quantity of prions thereon into a sterilization chamber (12);

lowering pressure in the sterilization chamber (12) to a pressure below the vapor pressure of hydrogen peroxide in the chamber (12);

vaporizing a solution comprising hydrogen peroxide and contacting the article with the hydrogen peroxide vapor, wherein the concentration of the hydrogen peroxide in the solution exceeds 70 percent;

exciting the hydrogen peroxide vapor into the plasma phase, wherein the plasma power density is at least 10 milliwatts/cm$^3$; and

inactivating prions in the chamber (12) through contact between the prions and the hydrogen peroxide vapor in the plasma phase.

2. A method according to claim 1 wherein the concentration of the hydrogen peroxide in the solution is more than 75 percent, preferably more than 80 percent, more preferably more than 85 percent and most preferably more than 90 percent.

3. A method according to claim 1 or claim 2, wherein the temperature of the article during the step of contacting the article with hydrogen peroxide vapor is more than 30 degrees Celsius, preferably more than 35 degrees Celsius, more preferably more than 40 degrees Celsius, and most preferably more than 45 degrees Celsius.

4. A method according to claim 1 wherein the plasma power density is at least 20 milliwatts/cm$^3$, preferably at least 30 milliwatts/cm$^3$, more preferably at least 40 milliwatts/cm$^3$, and most preferably at least 50 milliwatts/cm$^3$.

5. A method according to claim 1 or claim 4, wherein the plasma is in contact with the prions for at least 2 minutes, preferably at least 3 minutes, and more preferably at least 4 minutes.

6. A method according to any of claims 1, 4 and 5, wherein the plasma is generated between an anode and a cathode and wherein the gap therebetween is less than 1.5 cm (0.6 inches), preferably 1.3 cm (0.5 inches) or less, and more preferably about 1.3 cm (0.5 inches).

7. A method according to any of claims 1, 4 and 5, wherein the plasma is generated between an anode and a cathode and wherein the minimum distance between the prions and the closer of the anode and cathode is 3.8 cm (1.5 inches), preferably 2.5 cm (one inch) or less.

8. A method according to claim 7 wherein the distance between the prions and the anode is 2.5 cm (one inch) or less.

9. A method according to any preceding claim in which the hydrogen peroxide effects sterilization of the article.

10. A method according to any preceding claim, comprising increasing the concentration of hydrogen peroxide in the vapor which contacts the prions, prior to contacting the prions therewith.

11. A method according to claim 10 wherein the step of increasing the concentration of the hydrogen peroxide is performed by selectively removing water from the solution and exhausting that water through the sterilization chamber (12).

12. A method according to any preceding claim, wherein the steps of steps of lowering pressure in the sterilization chamber (12) and admitting the solution into the sterilization chamber (12) comprise a cycle and wherein the cycle is repeated, preferably more than once.

13. A method according to any preceding claim, wherein during the step of contacting the article with hydrogen peroxide vapor, the vapor has a concentration of 10 mg/L or more, preferably 15 mg/L or more, more preferably 20 mg/L or more, and most preferably 25 mg/L or more.

14. A method according to any preceding claim, further comprising the step of condensing a portion of the hydrogen peroxide vapor onto the article and wherein the concentration of that condensed hydrogen peroxide is at least 80% hydrogen peroxide by weight, preferably at least 85% hydrogen peroxide by weight, more preferably at least 90% hydrogen peroxide by weight, and most preferably at least 95% hydrogen peroxide by weight.

15. A method according to claim 14 and further comprising the step of evaporating at least a portion of that condensed hydrogen peroxide by pumping the sterilization chamber (12) to a lower pressure.

**Patentansprüche**

1. Verfahren zum Inaktivieren von Prionen, umfassend die Schritte:

   Platzieren eines Gegenstands, der eine Menge an Prionen darauf aufweist, in eine Sterilisationskammer (12);
   Absenken des Drucks in der Sterilisationskammer (12) auf einen Druck unterhalb des Dampfdrucks von Wassersoffperoxid in der Kammer (12) ;
   Verdampfen einer Lösung, die Wasserstoffperoxid umfasst, und Kontaktieren des Gegenstands mit dem Wasserstoffperoxiddampf, wobei die Konzentration des Wasserstoffperoxids in der Lösung 70 Prozent übersteigt;
   Anregen des Wasserstoffperoxiddampfs in die Plasmaphase, wobei die Plasma-Leistungsdichte wenigstens 10 Milliwatt/cm$^3$ beträgt; und
   Inaktivieren von Prionen in der Kammer (12) durch Kontakt zwischen den Prionen und dem Wasserstoffperoxiddampf in der Plasmaphase.

2. Verfahren gemäß Anspruch 1, wobei die Konzentration des Wasserstoffperoxids in der Lösung mehr als 75 Prozent beträgt, vorzugsweise mehr als 80 Prozent, bevorzugter mehr als 85 Prozent, höchst bevorzugt mehr als 90 Prozent.

3. Verfahren gemäß Anspruch 1 oder Anspruch 2, wobei die Temperatur des Gegenstands während des Schritts des Kontaktierens des Gegenstands mit Wasserstoffperoxiddampf mehr als 30 Grad Celsius beträgt, vorzugsweise mehr als 35 Grad Celsius, bevorzugter mehr als 40 Grad Celsius, höchst bevorzugt mehr als 45 Grad Celsius.

4. Verfahren gemäß Anspruch 1, wobei die Plasma-Leistungsdichte wenigstens 20 Milliwatt/cm$^3$ beträgt, vorzugsweise wenigstens 30 Milliwatt/cm$^3$, bevorzugter wenigstens 40 Milliwatt/cm$^3$, höchst bevorzugt wenigstens 50 Milliwatt/cm$^3$.

5. Verfahren gemäß Anspruch 1 oder Anspruch 4, wobei das Plasma wenigstens 2 Minuten in Kontakt mit den Prionen steht, vorzugsweise wenigstens 3 Minuten, bevorzugter wenigstens 4 Minuten.

6. Verfahren gemäß einem der Ansprüche 1, 4 und 5, wobei das Plasma zwischen einer Anode und einer Kathode erzeugt wird und wobei der Abstand dazwischen weniger als 1,5 cm (0,6 Inch) beträgt, vorzugsweise 1,3 cm (0,5 Inch) oder weniger, bevorzugter etwa 1,3 cm (0,5 Inch).

7. Verfahren gemäß einem der Ansprüche 1, 4 und 5, wobei das Plasma zwischen einer Anode und einer Kathode erzeugt wird und wobei der Mindestabstand zwischen den Prionen und dem Näheren von der Anode und der Kathode 3,8 cm (1,5 Inch) beträgt, vorzugsweise 2,5 cm (ein Inch) oder weniger.

8. Verfahren gemäß Anspruch 7, wobei der Abstand zwischen den Prionen und der Anode 2,5 cm (ein Inch) oder weniger beträgt.

9. Verfahren gemäß einem der vorstehenden Ansprüche, wobei das Wasserstoffperoxid Sterilisation des Gegenstands bewirkt.

10. Verfahren gemäß einem der vorstehenden Ansprüche, umfassend das Erhöhen der Konzentration von Wasserstoffperoxid in dem Dampf, der die Prionen kontaktiert, vor dem Kontaktieren der Prionen damit.

11. Verfahren gemäß Anspruch 10, wobei der Schritt des Erhöhens der Konzentration des Wasserstoffperoxids durch selektives Entfernen von Wasser aus der Lösung und Ausstoßen dieses Wassers durch die Sterilisationskammer (12) durchgeführt wird.

12. Verfahren gemäß einem der vorstehenden Ansprüche, wobei die Schritte der Schritte des Absenkens des Drucks in der Sterilisationskammer (12) und des Zuführens der Lösung in die Sterilisationskammer (12) einen Zyklus bilden und wobei der Zyklus wiederholt wird, vorzugsweise mehr als einmal.

13. Verfahren gemäß einem der vorstehenden Ansprüche, wobei während des Schritts des Kontaktierens des Gegenstands mit Wasserstoffperoxiddampf der Dampf eine Konzentration von 10 mg/l oder mehr aufweist, vorzugsweise 15 mg/l oder mehr, bevorzugter 20 mg/l oder mehr, höchst bevorzugt 25 mg/l oder mehr.

14. Verfahren gemäß einem der vorstehenden Ansprüche, ferner umfassend den Schritt des Kondensierens eines Teils des Wasserstoffperoxiddampfs auf dem Gegenstand und wobei die Konzentration des kondensierten Wasserstoffperoxids wenigstens 80 Gew.-% Wasserstoffperoxid beträgt, vorzugsweise wenigstens 85 Gew.-% Wasserstoffperoxid, bevorzugter wenigstens 90 Gew.-% Wasserstoffperoxid, höchst bevorzugt wenigstens 95 Gew.-% Wasserstoffperoxid.

15. Verfahren gemäß Anspruch 14, ferner umfassend den Schritt des Verdampfens von wenigstens einem Teil des kondensierten Wasserstoffperoxids durch Pumpen der Sterilisationskammer (12) auf einen tieferen Druck.

**Revendications**

1. Procédé d'inactivation de prions comprenant les étapes consistant à :

mettre un article ayant une certaine quantité de prions sur lui dans une chambre de stérilisation (12) ;
abaisser la pression dans la chambre de stérilisation (12) à une pression au-dessous de la pression de vapeur du peroxyde d'hydrogène dans la chambre (12) ;
vaporiser une solution comprenant du peroxyde d'hydrogène et mettre en contact l'article avec la vapeur de peroxyde d'hydrogène, la concentration du peroxyde d'hydrogène dans la solution dépassant 70 pour cent ;
exciter la vapeur de peroxyde d'hydrogène en une phase plasma, la densité de puissance du plasma étant d'au moins 10 milliwatts/cm$^3$ ; et
inactiver les prions dans la chambre (12) grâce au contact entre les prions et la vapeur de peroxyde d'hydrogène en phase plasma.

2. Procédé selon la revendication 1 dans lequel la concentration du peroxyde d'hydrogène dans la solution est supérieure à 75 pour cent, de préférence supérieure à 80 pour cent, plus préférablement supérieure à 85 pour cent et de préférence par-dessus tout supérieure à 90 pour cent.

3. Procédé selon la revendication 1 ou la revendication 2, dans lequel la température de l'article pendant l'étape consistant à mettre en contact l'article avec de la vapeur de peroxyde d'hydrogène est supérieure à 30 degrés Celsius, de préférence supérieure à 35 degrés Celsius, plus préférablement supérieure à 40 degrés Celsius et de préférence par-dessus tout supérieure à 45 degrés Celsius.

4. Procédé selon la revendication 1 dans lequel la densité de puissance du plasma est d'au moins 20 milliwatts/cm$^3$, de préférence d'au moins 30 milliwatts/cm$^3$, plus préférablement d'au moins 40 milliwatts/cm$^3$ et de préférence par-dessus tout d'au moins 50 milliwatts/cm$^3$.

5. Procédé selon la revendication 1 ou la revendication 4, dans lequel le plasma est en contact avec les prions pendant au moins 2 minutes, de préférence au moins 3 minutes et plus préférablement au moins 4 minutes.

6. Procédé selon l'une quelconque des revendications 1, 4 et 5, dans lequel le plasma est généré entre une anode et une cathode et dans lequel l'espace entre celles-ci est inférieur à 1,5 cm (0,6 pouce), de préférence inférieur ou égal à 1,3 cm (0,5 pouce) et plus préférablement d'environ 1,3 cm (0,5 pouce).

7. Procédé selon l'une quelconque des revendications 1, 4 et 5, dans lequel le plasma est généré entre une anode et une cathode et dans lequel la distance minimale entre les prions et la plus proche de l'anode et de la cathode est inférieure ou égale à 3,8 cm (1,5 pouce), de préférence à 2,5 cm (un pouce).

8. Procédé selon la revendication 7 dans lequel la distance entre les prions et l'anode est inférieure ou égale à 2,5 cm (un pouce).

9. Procédé selon l'une quelconque des revendications précédentes dans lequel le peroxyde d'hydrogène effectue la stérilisation de l'article.

10. Procédé selon l'une quelconque des revendications précédentes, comprenant l'augmentation de la concentration en peroxyde d'hydrogène dans la vapeur qui vient en contact avec les prions, avant la mise en contact des prions avec celle-ci.

11. Procédé selon la revendication 10 dans lequel l'étape d'augmentation de la concentration du peroxyde d'hydrogène est effectuée par élimination sélective d'eau de la solution et évacuation de cette eau par la chambre de stérilisation (12).

**12.** Procédé selon l'une quelconque des revendications précédentes, dans lequel les étapes consistant à abaisser la pression dans la chambre de stérilisation (12) et faire entrer la solution dans la chambre de stérilisation (12) comprennent un cycle et dans lequel le cycle est répété, de préférence plus d'une fois.

**13.** Procédé selon l'une quelconque des revendications précédentes, dans lequel pendant l'étape consistant à mettre en contact l'article avec de la vapeur de peroxyde d'hydrogène, la vapeur a une concentration supérieure ou égale à 10 mg/l, de préférence supérieure ou égale à 15 mg/l, plus préférablement supérieure ou égale à 20 mg/l et de préférence par-dessus tout supérieure ou égale à 25 mg/l.

**14.** Procédé selon l'une quelconque des revendications précédentes, comprenant en outre l'étape consistant à condenser une partie de la vapeur de peroxyde d'hydrogène sur l'article et dans lequel la concentration de ce peroxyde d'hydrogène condensé est d'au moins 80 % de peroxyde d'hydrogène en poids, de préférence d'au moins 85 % de peroxyde d'hydrogène en poids, plus préférablement d'au moins 90 % de peroxyde d'hydrogène en poids et de préférence par-dessus tout d'au moins 95 % de peroxyde d'hydrogène en poids.

**15.** Procédé selon la revendication 14 et comprenant en outre l'étape consistant à faire évaporer au moins une partie de ce peroxyde d'hydrogène condensé par pompage de la chambre de stérilisation (12) à une plus faible pression.

FIG. 1

EP 2 279 761 B1

# FIG. 2

# FIG. 3

EP 2 279 761 B1

# FIG. 3A

EP 2 279 761 B1

# FIG. 3B

# FIG. 4

FIG. 5

## FIG. 6

# FIG. 7

EP 2 279 761 B1

62c

8

8

66c

102

100

78

92c

## FIG. 8

# FIG. 9

EP 2 279 761 B1

# FIG. 10

# FIG. 11

# FIG. 12

FIG. 13

# FIG. 14

EP 2 279 761 B1

# FIG. 14A

EP 2 279 761 B1

FIG. 15

FIG. 16

# FIG. 17

EP 2 279 761 B1

# FIG. 18

EP 2 279 761 B1

# FIG. 19

EP 2 279 761 B1

# FIG. 20

412

408

410

414

404

FIG. 21

FIG. 22

FIG. 23

Flow chart for calculating concentration of condensed peroxide

Known parameters: Chamber temperature & size,
Peroxide liquid concentration & injection volume

| Step 1 | Calculate density of liquid $H_2O_2$ (From liquid concentration, temperature & physical property data) |
|---|---|

| Step 2 | Calculate the total number moles of $H_2O$ and $H_2O_2$ (From liquid concentration, injection volume, density & molecular |
|---|---|

| Step 3A | (With non-condensing case) Calculate the total vapor pressure ($H_2O + H_2O_2$) using ideal gas law | | Step 3B | (With condensing case) Calculate the total vapor pressure using the vapor pressure equilibrium data and an arbitrarily assumed liquid phase composition |
|---|---|---|---|---|

| Step 4 | Determine total vapor pressure by taking the smaller of the above two |
|---|---|

| Step 5 | Calculate the number of moles in the vapor phase (with ideal gas law based on step 4) |
|---|---|

| Step 6 | Calculate the number of moles in the liquid phase (difference between steps 2 and 5) |
|---|---|

| Step 7 | Calculate vapor composition (from the vapor composition equilibrium data using the assumed liquid composition in step 3B) |
|---|---|

| Step 8 | Calculate # of moles of $H_2O_2$ in each phase (with total number of moles in each phase and the compositions in each phase) |
|---|---|

| Step 9 | Calculate total number of moles of $H_2O_2$ (Sum of peroxide in liquid and vapor phases) |
|---|---|

| Step 10 | Compare the calculated total number of moles of $H_2O_2$ from step 9 to the total number of moles of $H_2O_2$ injected |
|---|---|

| Step 11 | If the difference exceeds the tolerance limit, a new value for liquid composition is calculated and the calculation process is repeated |
|---|---|

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03031551 A
- US 2006008494 A
- US 6106772 A, Kohler
- US 72897300 A, Nguyen
- US 4643867 A, Jacobs
- US 20020068012 A, Platt, Jr.

- US 6365102 B, Wu
- US 20040262146 A
- US 5851485 A, Lin
- US 6379631 B, Wu
- US 4704254 A, Nichols